# EUROPÄISCHE PATENTSCHRIFT

(11) **EP 0 476 292 B1**
(45) Veröffentlichungstag und Bekanntmachung des Hinweises auf die Patenterteilung: **02.01.1997**
(21) Anmeldenummer: 91113255.3
(22) Anmeldetag: 07.08.1991
(51) Int. Cl.: A61L 11/00

(54) **Mobile Desinfektionsanlage**
Mobile disinfection installation
Installation mobile de désinfection

(30) Priorität: 13.09.1990 DE 9013046 U
(43) Veröffentlichungstag der Anmeldung: 25.03.1992
(73) Patentinhaber: DEUTSCHE BABCOCK ANLAGEN GMBH, 46003 Oberhausen (DE)
(72) Erfinder: Noetzel, Hans, W-3000 Hannover 91 (DE)
(74) Vertreter: Planker, Karl-Josef, Dipl.-Phys.

(56) Entgegenhaltungen:
- EP-A- 0 277 507
- EP-A- 0 372 664
- DE-A- 2 505 185
- DE-U- 8 816 225

## Beschreibung

Die Erfindung betrifft eine mobile Desinfektionsanlage für infektiösen Abfall, insbesondere Krankenhausabfall, gemäß dem Oberbegriff des Patentanspruchs 1.

Die Beseitigung des in Krankenhäusern anfallenden infektiösen Abfalls erfolgt bisher meistens in besonderen Verbrennungsanlagen. Da der Transport von derartigen kontaminierten Abfällen auf öffentlichen Verkehrswegen erheblichen Einschränkungen und Auflagen unterliegt, sind die bestehenden Anlagen durchweg im Krankenhausgelände aufgestellt. Die teils schon jahrzehntealten Verbrennungsanlagen sind durch sehr hohe Betriebskosten gekennzeichnet und genügen vielfach nicht im entferntesten den modernen gesetzlichen Vorschriften auf dem Gebiet des Emissionsschutzes.

Um Kosten einzusparen und Belästigungen und Gefährdungen der Umwelt zu vermeiden, hat man schon vereinzelt Desinfektionsanlagen eingesetzt, in denen der Krankenhausmüll so vorbehandelt wird, daß er anschließend gefahrlos wie gewöhnlicher Hausmüll transportiert und weiterbehandelt, insbesondere auch zusammen mit dem Hausmüll deponiert oder verbrannt werden kann. Zwecks weiterer Kostensenkung hat man auch schon fahrbare Desinfektionsanlagen vorgeschlagen, die eine Anzahl von Krankenhäusern reihum bedienen.

Eine Anlage gemäß dem Oberbegriff des Patentanspruchs 1 ist durch die EP-A2-0 277 507 bekannt geworden. Bei dieser Anlage bildet der mit einer Mantelheizung versehene Schneckenförderer die Desinfektionsstrecke. Ein Gemisch von Luft und Wasserdampf wird am Ende des Schneckenförderers abgesaugt und über einen Aktivkohlefilter ins Freie ausgestoßen. Die Tragkonstruktion kann als Auflieger eines Sattelschleppers ausgebildet sein.

Bei einem Schneckenförderer bildet das Material während des Transportes eine lockere Schüttung, die nur schwach umgewälzt wird. Partikel, die sich im Inneren der Schüttung befinden, haben keinen intensiven Kontakt mit dem Behandlungsgas und werden durch umgebendes Material gegen die heißen Wandflächen abgeschirmt. Dieser Nachteil wirkt sich umso stärker aus, je größer der Schüttungsquerschnitt ist. Eine gleichmäßige Erhitzung aller Partikel läßt sich nur bei relativ kleinen Querschnitten und langen Verweilzeiten gewährleisten. Dadurch ist die Durchsatzleistung beschränkt. Gerade bei mobilen Anlagen hängt aber die Wirtschaftlichkeit davon ab, daß an jedem Standort der angesammelte Abfall in möglichst kurzer Zeit desinfiziert wird, damit möglichst viele verschiedene Standorte bedient werden können.

Aus der DE-PS 37 05 364 ist eine andere Desinfektionsanlage bekannt, bei der die Desinfektionsstrecke ebenfalls als Schneckenförderer ausgebildet ist. Sie unterscheidet sich von der zuerst erwähnten Anlage unter anderem dadurch, daß das gasförmige Behandlungsmedium in geschlossenem Kreis geführt wird, wobei es sich innerhalb des Schneckenförderers im Gleichstrom mit dem zu behandelnden Abfallmaterial bewegt.

Aus der DE-OS 25 05 185 ist es bekannt, infektiösen Abfall kontinuierlich unter Heißluft- oder Keißdampf-Atmosphäre in einem Drehrohr zu desinfizieren. Vor oder während der Desinfektion soll eine Zerkleinerung erfolgen. Zur Volumenverminderung wird empfohlen, eine Presse nachzuschalten.

Der Erfindung liegt die Aufgabe zugrunde, eine Anlage der im Oberbegriff des Patentanspruchs 1 angegebenen Gattung zu schaffen, die bei hohem Durchsatz eine gleichmäßige, intensive Erhitzung und somit eine sichere Desinfektion gewährleistet.

Diese Aufgabe wird durch die kennzeichnenden Merkmale des Anspruchs 1 gelöst.

Bei der erfindungsgemäßen mobilen Anlage erfolgt die eigentliche Desinfektion in dem Drehrohr, das im Vergleich zum Schneckenförderer einen großen Querschnitt hat. Dadurch kann unter den beengten Raumverhältnissen einer mobilen Anlage eine große Durchsatzleistung erzielt werden. Infolge der Drehbewegung werden fortwährend Teilmengen des Trommelinhaltes angehoben und wieder abgeworfen, so daß sich ein Rieselschleier von Partikeln bildet, die intensiv von dem Behandlungsgas umströmt werden. Der ansteigende Schneckenförderer überbrückt als Zwischenförderer den Höhenunterschied zwischen dem Fuß des Beschickungsschachtes und der Aufgabeöffnung des Drehrohrs. Er ermöglicht auf diese Weise die Kombination des Beschickungsschachtes, der praktisch die volle für den Straßentransport zulässige Höhe in Anspruch nimmt, mit dem Drehrohr, dessen Aufgabeöffnung sich wegen des relativ großen Durchmessers und mit Rücksicht auf den am Drehrohrende befindlichen Materialaustrag zwangsläufig in einer gewissen Höhe über dem Boden befindet.

Natürlich findet in dem Schneckenförderer schon eine Vorwärmung statt. Das großvolumige Austraggehäuse wirkt für den abschließenden Gasstrom als Beruhigungskammer, in der mitgeführte Feststoff-Partikel - zumindest gröbere Partikel - abgeschieden werden.

Durch den nach Art einer Schleuse zu betätigenden Plattenschieber gemäß Anspruch 2 wird die Einfüllkammer, der die zu desinfizierenden Abfälle in geschlossenen Gebinden zugeführt werden, von infektiösen Partikeln völlig freigehalten. Dadurch wird eine hygienisch einwandfreie Beschickung gewährleistet.

Die in Förderrichtung zunehmende Steigung der Schneckengänge gemäß Anspruch 3 bewirkt eine Auflockerung des Materials.

Durch das Merkmal des Anspruchs 4 wird schon am Anfang des Drehrohrs ein den gesamten Querschnitt ausfüllender Strom des Behandlungsgases erzeugt. Durch die neuartige Anordnung der Öffnungen wird sichergestellt, daß nach Beendigung des Beschickungsvorganges der Trommelmantel auch in unmittelbarer Nähe der aufgabeseitigen Stirnwand freigeblasen wird, so daß Ablagerungen unter der Einmündung des Schneckenförderers vermieden werden.

Gemäß Anspruch 5 läßt sich der Behandlungsgasstrom an die Materialverteilung im Rieselschleier anpassen.

Die Merkmale der Ansprüche 6 und 7 erlauben eine Beeinflussung der Verweilzeit.

Durch das Merkmal des Anspruchs 8 wird die Aufrechterhaltung der erforderlichen Temperatur gewährleistet. Die Abstrahlung von Wärme wird weitgehend vermieden.

Das Merkmal des Anspruchs 9 ermöglicht eine weitere Verbesserung der Temperatursteuerung.

Durch das Merkmal des Anspruchs 10 wird der Austritt infektiös belasteter oder übelriechender Gase völlig vermieden.

Die Bypaßleitung gemäß Anspruch 11 erlaubt es, große Gasmengen über die Vorlagekammer und unter Umgehung des Schneidwerkes über den Schneckenförderer zu leiten.

Eine bevorzugte Ausführungsform der mobilen Anlage ist in Anspruch 12 angegeben.

Die Anordnung gemäß Anspruch 13 ist im Hinblick auf die Beschickung besonders vorteilhaft.

Durch das Merkmal des Anspruchs 14 wird die nutzbare Bauhöhe für den Beschickungsschacht vergrößert.

Mit Hilfe der Austragschnecke gemäß Anspruch 15 wird das Volumen des desinfizierten Abfalls durch Kompression verkleinert und sein Gewicht durch Auspressen von Wasser vermindert. Das in der Schnecke befindliche Material bewirkt eine zuverlässige Abdichtung gegen den Austritt von Behandlungsgas.

Dank der Ausbildung gemäß Anspruch 16 läßt sich die Austragschnecke den betrieblichen Anforderungen optimal anpassen.

Gemäß Anspruch 17 wird der Bereich des Aufliegers, in dem die nutzbare Bauhöhe am stärksten beschränkt ist, für die Unterbringung des modulartig zusammengefaßten Versorgungsteils genutzt.

Das Merkmal des Anspruchs 18 gewährleistet zusätzliche Sicherheit gegen umweltschädigende Emissionen.

Gemäß den Ansprüchen 19 und 20 werden Verunreinigungen, die in die Einfüllkammmer bzw. in das Innere des Gehäuses gelangt sind, mit der Verbrennungsluft angesaugt und verbrannt.

Die Zeichnung dient zur Erläuterung der Erfindung anhand eines teilweise vereinfacht dargestellten Ausführungsbeispiels.

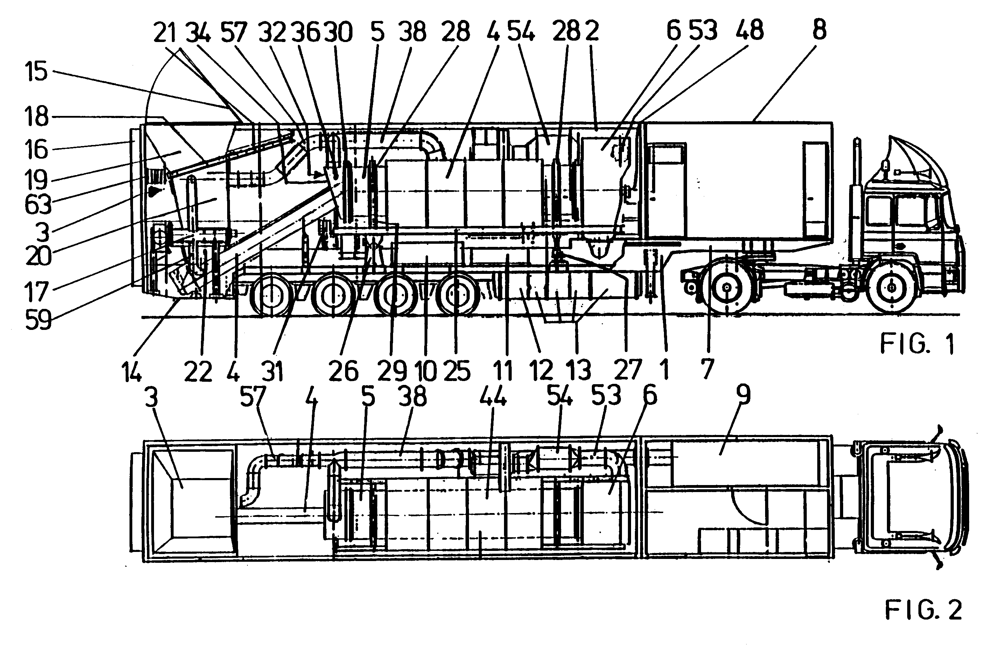

Figur 1 zeigt maßstabsgetreu eine Seitenansicht einer mobilen Desinfektionsanlage.

Figur 2 zeigt eine Draufsicht der Anlage.

Figur 3 zeigt eine schematische Darstellung.

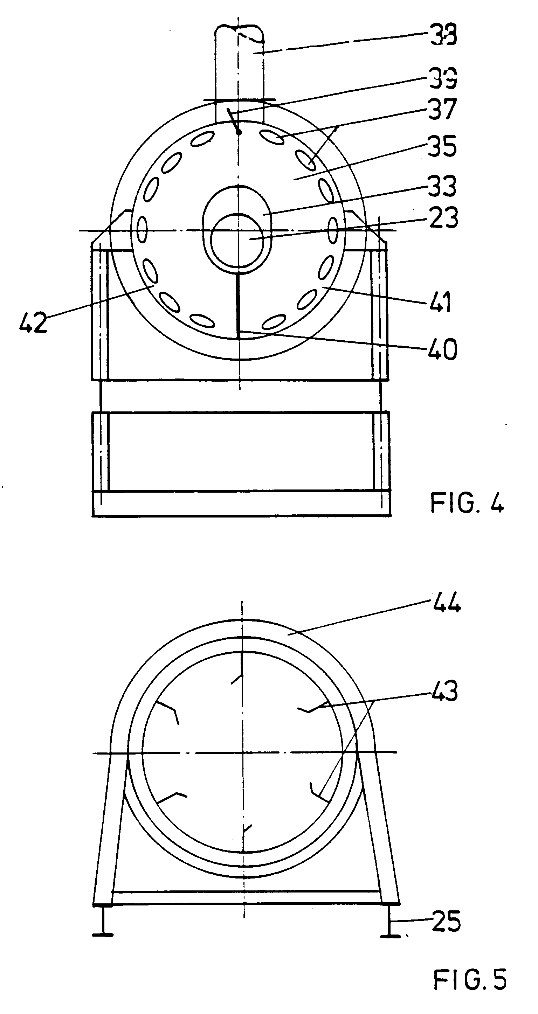

Figur 4 zeigt das Eintraggehäuse mit der aufgabeseitigen Stirnwand.

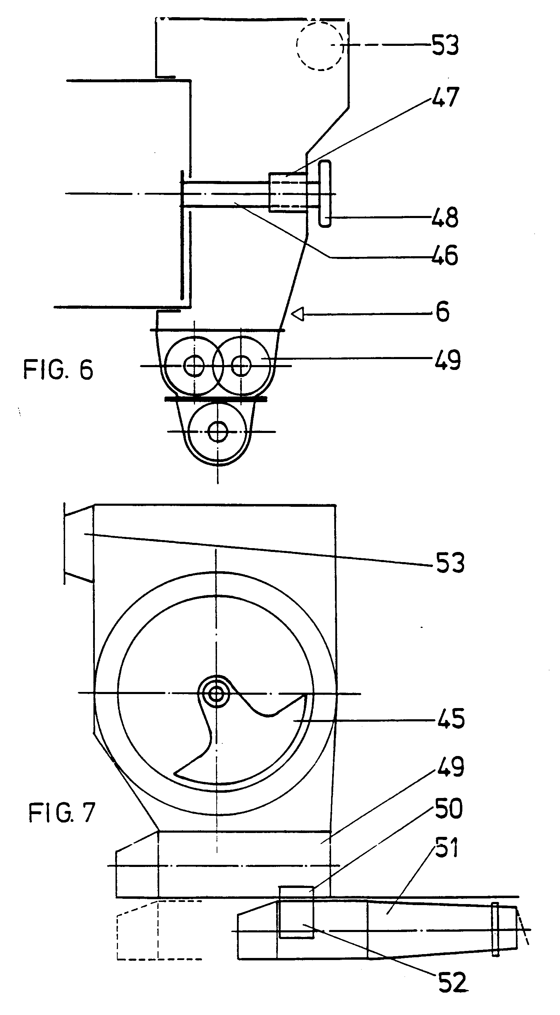

Figur 5 zeigt einen Schnitt durch das Drehrohr.

Figur 6 zeigt das Ausfallende des Drehrohres mit dem Ausfallgehäuse von der Seite.

Figur 7 zeigt das Ausfallende des Drehrohres mit dem Ausfallgehäuse, in Richtung der Achse des Drehrohres gesehen.

Auf dem als Tragkonstruktion dienenden Auflieger 1 eines Sattelschleppers ist ein Gehäuse 2 montiert, dessen Boden als Wanne ausgebildet und im Heckbereich nach unten gezogen ist. Das Gehäuse 2 nimmt die eigentliche Desinfektionsanlage auf, d.h. insbesondere diejenigen Komponenten, die im Betrieb von dem zu desinfizierenden Abfall durchlaufen werden, und zwar einen Beschickungsschacht 3, einen Schneckenförderer 4, ein Drehrohr 5 und ein Ausfallgehäuse 6. Auf dem Schwanenhals 7 des Aufliegers 1 ist in einem separaten Gehäuse 8 ein Energieversorgungsaggregat 9 untergebracht, welches insbesondere einen Brenner, einen Dampferzeuger, einen Erhitzer für das Behandlungsmedium, einen Wärmeaustauscher für Thermoöl sowie Schalt- und Bedienungsorgane umfaßt. Unter dem Drehrohr 5 liegen Tanks 10, 11 für Heizöl und Thermoöl. An der Unterseite des Aufliegers 1 ist ein Kompressor 12 sowie Behälter 13 für Abwasser, Desinfektionsmittel und Frischwasser aufgehängt.

Der Beschickungsschacht 3, der sich über die volle Bauhöhe erstreckt und sich trichterartig nach unten verengt, ist am Heck des Aufliegers 1 über dem nach unten gezogenen Bodenteil 14 angeordnet. Er ist durch eine Klappe 15 verschließbar. Auf seiner Hinterseite ist eine Hub-Kipp-Vorrichtung 16 für die Container angebracht, in denen üblicherweise der infektiöse Abfall gesammelt wird. Im unteren Teil des Beschickungsschachtes 3 sitzt ein Schneidwerk 17. Der über dem Schneidwerk 17 befindliche Teil des Beschickungsschachtes 3 ist durch einen Plattenschieber 18, dessen Führung nach hinten schräg abwärts gerichtet ist, in eine Einfüllkammer 19 und eine Vorlagekammer 20 unterteilbar. Ein gasdichter Schieberkasten 21 ist an der Vorderseite des Beschickungsschachtes 3 befestigt. Unter dem Schneidwerk 17 befindet sich ein Sammeltrichter 22, der am Fuß des Beschickungsschachtes 3 in den Schneckenförderer 4 einmündet.

Dieser besteht im wesentlichen aus einer Schnecke 23, deren Steigung in Förderrichtung zunimmt, und einem ovalen Schneckentrog 24. Der Schneckenförderer 4 ist unter einem Winkel von etwa 30° schräg nach oben gerichtet und mündet in das nachgeschaltete Drehrohr 5 ein.

Das Drehrohr 5 ist auf einem Kipprahmen 25 gelagert, der aufgabeseitig durch gelenkige Stützen 26 und ausfallseitig durch höhenverstellbare Stützen 27 mit dem Auflieger 1 verbunden ist. Es ist mit zwei Laufkränzen 28 versehen, die sich auf vier Laufrollen abstützen, deren Lagerböcke mit dem Kipprahmen 25 verschraubt sind. Der Antrieb erfolgt über einen Zahnring 30 durch einen Getriebemotor 31.

Fest mit dem Kipprahmen 25 verbunden ist ein Eintraggehäuse 32 mit einem ovalen, dem Querschnitt des Schneckentroges 24 angepaßten Durchlaß 33, der die Einmündung des Schneckenförderers in das Drehrohr 5 bildet. Mit der hinteren Stirnwand 34 des Eintraggehäuses 32 ist der Schneckentrog 24 durch einen in der Zeichnung nicht erkennbaren Kompensator verbunden. Die Stirnwand 34 ist schräg gestellt, so daß sie mit der gegenüberliegenden senkrechten Stirnwand 35 eine sich nach unten keilförmig verengende, ringförmige Verteilerkammer 36 einschließt. Die Stirnwand 35 schließt das Drehrohr 5 aufgabeseitig ab. Sie ist mit einem Kranz von Öffnungen 37 für ein gasförmiges Behandlungsmedium versehen. Von oben mündet ein Zuleitungsrohr 38 in die Verteilerkammer 36 ein. Im Bereich der Einmündung ist eine Verteilerklappe 39 angeordnet. Gegenüber der Einmündung, d.h. unter dem Durchlaß 33, ist in der Verteilerkammer 36 eine Trennwand 40 angeordnet. Dadurch wird die Verteilerkammer in zwei von der Einmündung ausgehende Zweige 41, 42 unterteilt.

Das Drehrohr 5 ist mit abgewinkelten Hubschaufeln 43 bestückt, die in mehreren in Längsrichtung aufeinanderfolgenden Kränzen - jeweils winkelversetzt zu den Hubschaufeln des benachbarten Kranzes - angeordnet sind. Es ist von einer fest mit dem Kipprahmen 25 verbundenen, in vier getrennt beheizbare Zonen unterteilten Haube 44 umgeben. Die Beheizung der Haube 44 erfolgt mit Thermoöl. Am Auslaufende des Drehrohres 5 befindet sich ein Stausegment 45. Es ist auf einer zum Drehrohr 5 koaxialen Welle 46 befestigt. Diese sitzt in einem Lager 47, das mit der vorderen Wand des Ausfallgehäuses 6 verbunden ist. Die Welle 46 ist samt Stausegment 45 mit einem nur angedeuteten Stellorgan 48 winkelverstellbar und in jeder Position arretierbar.

Das Ausfallgehäuse 6 ist fest mit dem Kipprahmen 25 verbunden. In seinem unteren Teil ist als Austragvorrichtung eine Doppelschnecke 49 mit Austragöffnung 50 vorgesehen. Unter der Doppelschnecke 49 ist eine Austragschnecke 51 mit degressiver Steigung und Gegendruckklappe angeordnet. Sie ist aus einer inaktiven Transportstellung, die in Figur 7 durch gestrichelte Linien angedeutet ist, in ihre Betriebsstellung seitlich ausfahrbar. In dieser Stellung fluchtet ihr Zuführschacht 52 mit der Austragöffnung 50.

An den oberen, als erweiterte Beruhigungskammer ausgebildeten Teil des Ausfallgehäuses 6 ist ein Absaugrohr 53 angeschlossen. Dieses gehört zu einer in geschlossenem Kreis geführten Leitung, die auch ein Feinstaubfilter 54, ein Gebläse 55, einen Erhitzer 56 und das in die Verteilerkammer 36 einmündende Zuleitungsrohr 38 sowie ein Abzweigrohr 57 umfaßt, welches das Zuleitungsrohr 38 mit einem Gaseinlaß 58 der Vorlagekammer 20 verbindet. Von der Vorlagekammer 20 geht eine Bypaßleitung 59 aus, die in den Schneckentrog 24 unmittelbar am Fuß des Beschickungsschachtes 3 einmündet.

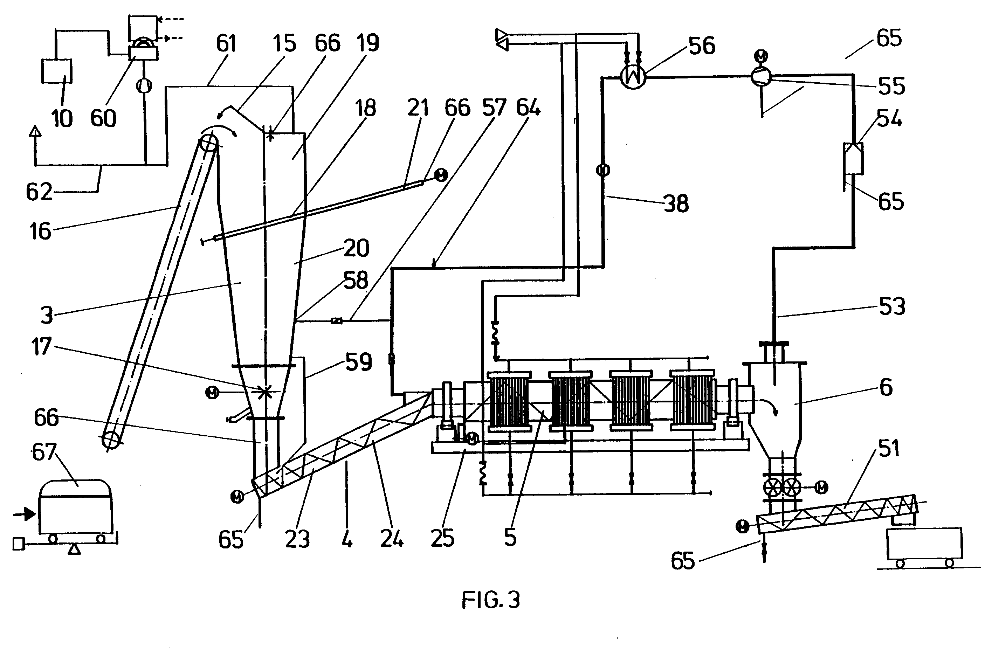

Der in dem Gehäuse 8 untergebrachte, nur in Figur 3 schematisch dargestellte Brenner 60 ist durch eine mit zwei Zweigen 61, 62 ausgestattete Luftansaugleitung einerseits mit der Einfüllkammer 19 des Beschickungsschachtes 3, andererseits mit dem Innenraum des Gehäuses 2 verbunden, das im Heckbereich eine Ansaugöffnung 63 aufweist.

Das Zuleitungsrohr 38 ist mit Anschlüssen 64 zum Eindüsen von Heißdampf versehen. An dem Schneckenförderer 4, der Austragschnecke 51, dem Feinstaubfilter 54 und dem Gebläse 55 sind - jeweils an der tiefsten Stelle - Abflußstutzen 65 für Schmutz- bzw. Kondenswasser angebracht, die über nicht dargestellte Leitungen mit dem Abwassertank verbunden sind. An verschiedenen Stellen des Beschickungsschachtes 3 sind Einspritzdüsen 66 für ein Desinfektionsmittel vorgesehen.

Die Anlage arbeitet folgendermaßen:
Der infektiöse, in Plastiksäcken oder Plastiktonnen verpackte Abfall wird in einem geschlossenen Container 67 an den Beschickungsschacht 3 herangefahren. Ausklappbare Traversen der Hub-Kipp-Vorrichtung 16 erfassen den Container 67 und heben ihn zunächst in eine Wartestellung. Er verbleibt dort solange, bis durch ein Signal die Freigabe zur Beschickung der Einfüllkammer 19 erfolgt. Die Klappe 15 wird erst geöffnet, nachdem der Plattenschieber 18 die Einfüllkammer 19 von der Vorlagekammer 20 getrennt hat. Der Inhalt des Containers 67 wird nun in die Einfüllkammer 19 entleert. Während des Einfüllvorganges wird in der Einfüllkammer 19 durch Absaugen von Luft über den Zweig 61 ein Unterdruck aufrechterhalten, so daß Emissionen verhindert werden.

Nachdem die Klappe 15 wieder geschlossen ist und die Vorlagekammer 20 leergefahren ist, wird der Plattenschieber 18 zurückgezogen, so daß der Inhalt der Einfüllkammer 19 in die Vorlagekammer 20 fällt und dem Schneidwerk 17 zugeführt wird. Nun erfolgt ein mehrfacher Luftwechsel der Einfüllkammer 19, indem ein Teil der Verbrennungsluft des Brenners 60 über die Einfüllkammer 19 angesaugt wird. Der zerkleinerte Abfall gelangt über den Schneckenförderer 4, in dem eine Auflockerung erfolgt, in das Drehrohr 5. Die Verweilzeit im Drehrohr 5 beträgt z.B. 20 Minuten, die Füllmenge bis zu 40 %. Das desinfizierte Material wird über die Doppelschnecke 49 und die Austragschnecke 51, in der das Volumen im Verhältnis 2:1 reduziert wird, einem Container 68 zugeführt.

Die Desinfektion erfolgt in dem Drehrohr 5 durch ein aus Luft und Wasserdampf bestehendes, auf 140 - 160 °C erhitztes Behandlungsmedium im Gleichstrom. Ein Teilstrom des Behandlungsmediums wird über das Abzweigrohr 57 in die Vorlagekammer 20 eingeleitet, gelangt teils über das Schneidwerk 17 und den Sammeltrichter 22 und teils über die Bypaßleitung 59 zum Schneckenförderer 4 und wird im Gleichstrom mit dem zerkleinerten und bereits vorgewärmten Abfall dem Drehrohr 5 zugeführt. Ein zweiter Teilstrom wird über das Zuleitungsrohr 38 und die beiden Zweige 41, 42 der Verteilerkammer durch die Öffnungen 37 direkt in das Drehrohr 5 eingeblasen. In dem großvolumigen Ausfallgehäuse wird das Behandlungsmedium anschließend vom Feststoff getrennt. Nachdem es in dem Feinstaubfilter 54 von Restpartikeln befreit worden ist, wird es über das Gebläse 55 dem Erhitzer 56 zugeführt, wo es wieder auf die vorgeschriebene Temperatur gebracht wird. Durch Eindüsen von Heißdampf über die Anschlüsse 64 wird die optimale Dampfkonzentration wieder hergestellt.

## Patentansprüche

1. Mobile Desinfektionsanlage für infektiösen Abfall, insbesondere Krankenhausabfall,
mit einer Tragkonstruktion,
mit einem Beschickungsschacht, der mindestens ein Schneidwerk und einen über dem Schneidwerk einmündenden Gaseinlaß aufweist,
und mit einem vom Fuß des Beschickungsschachtes ausgehenden, schräg ansteigenden Schneckenförderer,
dadurch gekennzeichnet, daß der Schneckenförderer (4) in ein mit Hubschaufeln (43) ausgestattetes Drehrohr (5) einmündet
und daß am Ausfallende des Drehrohres (5) ein geschlossenes Ausfallgehäuse (6) mit einer Austragvorrichtung (49 bis 52) für den desinfizierten Abfall und einem Absaugrohr (53) für das Behandlungsmedium angeordnet ist.

2. Anlage nach Anspruch 1, dadurch gekennzeichnet, daß der Innenraum des Beschickungsschachtes (3) oberhalb des Schneidwerkes (17) durch einen Plattenschieber (18) in eine Einfüllkammer (19) und eine Vorlagekammer (20) unterteilbar ist und daß der Gaseinlaß (58) in der Wand der Vorlagekammer (20) angeordnet ist.

3. Anlage nach Anspruch 1 oder Anspruch 2, dadurch gekennzeichnet, daß die Förderschnecke (23) eine progressive Steigung aufweist.

4. Anlage nach einem der Ansprüche 1 bis 3, gekennzeichnet durch einen den einmündenden Schneckenförderer (4) umgebendes Eintraggehäuse (32), dessen Stirnwand (35) das Drehrohr (5) aufgabeseitig abschließt und einen Kranz von Öffnungen (37) für ein gasförmiges Behandlungsmedium aufweist.

5. Anlage nach Anspruch 4, dadurch gekennzeichnet, daß der Innenraum des Eintraggehäuses (32) als Verteilerkammer (36) mit zwei von der Einmündung eines Zuleitungsrohres (38) ausgehenden Zweigen (41, 42) ausgebildet ist und daß im Bereich der Einmündung eine Verteilerklappe (39) angeordnet ist.

6. Anlage nach einem der Ansprüche 1 bis 5, gekennzeichnet durch höhenverstellbare Stützen (27) zum Verstellen des Neigungswinkels des Drehrohres (5).

7. Anlage nach einem der Ansprüche 1 bis 6, gekennzeichnet durch eine verstellbare Stauvorrichtung (45 bis 48) am Ausfallende des Drehrohres (5).

8. Anlage nach einem der Ansprüche 1 bis 7, dadurch gekennzeichnet, daß das Drehrohr (5) mit einer heizbaren Haube (44) versehen ist.

9. Anlage nach Anspruch 8, dadurch gekennzeichnet, daß die Haube (44) in mehrere Zonen unterteilt ist.

10. Anlage nach einem der Ansprüche 1 bis 9, gekennzeichnet durch eine in geschlossenem Kreis geführte Leitung, die das Absaugrohr (53), das Zuleitungsrohr (38), ein das Zuleitungsrohr (38) mit dem Gaseinlaß (58) verbindendes Abzweigrohr (57), ein Gebläse (55) und einen Erhitzer (56) für das Behandlungsgas umfaßt.

11. Anlage nach Anspruch 10, gekennzeichnet durch eine Bypaßleitung (59), die das Schneidwerk (17) überbrückt.

12. Anlage nach einem der Ansprüche 1 bis 11, dadurch gekennzeichnet, daß die Tragkonstruktion als Auflieger (1) eines Sattelschleppers ausgebildet ist.

13. Anlage nach Anspruch 12, dadurch gekennzeichnet, daß der Beschickungsschacht (3) am Heck des Aufliegers angeordnet ist und daß auf seiner Hinterseite eine Hub-Kipp-Vorrichtung (16) angebracht ist.

14. Anlage nach Anspruch 13, dadurch gekennzeichnet, daß der Auflieger (1) im Heckbereich einen nach unten gezogenen Bodenteil (14) aufweist.

15. Anlage nach einem der Ansprüche 1 bis 14, dadurch gekennzeichnet, daß die Austragvorrichtung des Ausfallgehäuses (6) mit einer Austragschnecke (51) mit degressiver Steigung und Gegendruckklappe und mit einer an der tiefsten Stelle angeordneten Abflußleitung (65) ausgestattet ist.

16. Anlage nach Anspruch 15, dadurch gekennzeichnet, daß die Austragschnecke (51) aus einer Transportstellung in eine Betriebsstellung seitlich ausfahrbar ist.

17. Anlage nach einem der Ansprüche 12 bis 16, dadurch gekennzeichnet, daß auf dem Schwanenhals (7) des Aufliegers (1) in einem separaten Gehäuse (8) ein Energieversorgungsaggregat angeordnet ist, welches einen Brenner (60), einen Dampferzeuger, einen Erhitzer (56) für das Behandlungsmedium, einen Wärmeaustauscher für Thermoöl sowie Schalt- und Bedienungselemente umfaßt.

18. Anlage nach einem der Ansprüche 1 bis 17, gekennzeichnet durch ein mit der Tragkonstruktion (1) verbundenes, die Desinfektionsanlage umschließendes Gehäuse (2), dessen Boden als Wanne ausgebildet ist.

19. Anlage nach einem der Ansprüche 1 bis 18, dadurch gekennzeichnet, daß der Brenner (60) durch eine Luftansaugleitung (61) mit der Einfüllkammer (19) verbunden ist.

20. Anlage nach Anspruche 18 oder 19, dadurch gekennzeichnet, daß der Brenner (60) durch eine Luftansaugleitung (62) mit dem Inneren des Gehäuses (2) verbunden ist.

## Claims

1. A mobile disinfecting unit for infectious waste, in particular hospital waste,
having a supporting structure,
having a feed chute which has at least one cutting mechanism and a gas inlet issuing above the cutting mechanism,
and having an upwards-inclined screw conveyor issuing from the base of the feed chute,
characterised in that the screw conveyor (4) issues into a rotary tube (5) provided with lifting vanes (43),
and in that a closed discharge housing (6) having a delivery device (49 to 52) for the disinfected waste and having a suction tube (53) for the treatment medium is arranged at the discharge end of the rotary tube (5).

2. A unit in accordance with Claim 1, characterised in that above the cutting mechanism (17), the interior space of the feed chute (3) can be divided into a filling chamber (19) and a receiving chamber (20) by means of a single-faced valve (18), and in that the gas inlet (58) is arranged in the wall of the storage chamber (20).

3. A unit in accordance with Claim 1 or Claim 2, characterised in that the conveyor screw (23) has a progressive inclination.

4. A unit in accordance with any one of Claims 1 to 3, characterised by a feed housing (32) which surrounds the issuing screw conveyor (4) and whose end wall (35) closes the rotary tube (5) at the infeed side and has a ring of openings (37) for a gaseous treatment medium.

5. A unit in accordance with Claim 4, characterised in that the interior space of the feed housing (32) is in the form of a distribution chamber (36) with two branches (41, 42) issuing from the mouth of an inlet pipe (38), and in that a distribution valve (39) is arranged in the region of the mouth.

6. A unit in accordance with any one of Claims 1 to 5, characterised by vertically-adjustable supports (27) for adjusting the angle of inclination of the rotary tube (5).

7. A unit in accordance with any one of Claims 1 to 6, characterised by an adjustable accumulation device (45 to 48) at the discharge end of the rotary tube (5).

8. A unit in accordance with any one of Claims 1 to 7, characterised in that the rotary tube (5) is provided with a heatable hood (44).

9. A unit in accordance with Claim 8, characterised in that the hood (44) is divided into a plurality of zones.

10. A unit in accordance with any one of Claims 1 to 9, characterised by a line which is guided in a closed circuit and comprises the suction tube (53), the inlet pipe (38), a branch pipe (57) connecting the inlet pipe (38) to the gas inlet (58), a fan (55) and a heater (56) for the treatment gas.

11. A unit in accordance with Claim 10, characterised by a by-pass line (59) which spans the cutting mechanism (17).

12. A unit in accordance with any one of Claims 1 to 11, characterised in that the supporting structure is in the form of a semitrailer (1) of a semitrailer train.

13. A unit in accordance with Claim 12, characterised in that the feed chute (3) is arranged at the rear of the semitrailer, and in that a lifting-tilting apparatus (16) is attached to its rear side.

14. A unit in accordance with Claim 13, characterised in that the semitrailer (1) has a downwards-lengthened bottom part (14) in the rear region.

15. A unit in accordance with any one of Claims 1 to 14, characterised in that the delivery device of the discharge housing (6) is provided with a delivery screw (51) with degressive inclination and back-pressure valve and with a discharge line (65) arranged at the lowest site.

16. A unit in accordance with Claim 15, characterised in that the delivery screw (51) is laterally extendable from a transportation position into an operational position.

17. A unit in accordance with any one of Claims 12 to 16, characterised in that an energy supply assembly is arranged - in a separate housing (8) - on the goose neck (7) of the semitrailer (1) and comprises a burner (60), a steam generator, a heater (56) for the treatment medium, a heat exchanger for thermo-oil, and control and operating elements.

18. A unit in accordance with any one of Claims 1 to 17, characterised by a housing (2) which is connected to the supporting structure (1), encloses the disinfecting unit and whose bottom is in the form of a hull.

19. A unit in accordance with any one of Claims 1 to 18, characterised in that the burner (60) is connected to the filling chamber (19) by means of an air intake line (61).

20. A unit in accordance with Claims 18 or 19, characterised in that the burner (60) is connected to the interior of the housing (2) by an air intake line (62).

## Revendications

1. Installation mobile de désinfection pour des déchets infectieux, en particulier des déchets d'hôpitaux, comprenant
une construction de support,
un puits de chargement, qui présente au moins un outil de coupe et une entrée de gaz débouchant au-dessus de l'outil de coupe, et
un convoyeur à vis disposé en oblique partant du pied du pied du puits de chargement,
caractérisée en ce que le convoyeur à vis (4) débouche dans un tube rotatif (5) qui est équipé de pales élévatrices (43), et
en ce que, à l'extrémité de sortie du tube rotatif (5), est agencé un corps de sortie (6) fermé, pourvu d'un dispositif d'évacuation (49 à 52) pour les déchets désinfectés et d'un tube d'aspiration (53) pour le milieu de traitement.

2. Installation suivant la revendication 1, caractérisée en ce que le volume interne du puits de chargement (3) peut être subdivisé au-dessus de l'outil de coupe (17) par un tiroir à plaques (18) en un compartiment d'introduction (19) et un compartiment de récolte (20) et en ce que l'entrée de gaz (58) est agencée dans la paroi du compartiment de récolte (20).

3. Installation suivant l'une des revendications 1 et 2, caractérisée en ce que la vis transporteuse (23) présente un pas progressif.

4. Installation suivant l'une des revendications 1 à 3, caractérisée par un corps d'entrée (32) qui entoure le convoyeur à vis (4) débouchant et dont la paroi frontale (35) ferme le tube rotatif (5) du côté alimentation et présente une couronne d'orifices (37) pour un milieu de traitement gazeux.

5. Installation suivant la revendication 4, caractérisée en ce que le volume interne du corps d'entrée (32) est réalisé sous la forme d'une chambre distributrice (36) avec deux embranchements (41, 42) partant de l'embouchure d'un tube d'alimentation (38) et en ce qu'un clapet de répartition (39) est agencé dans la zone de l'embouchure.

6. Installation suivant l'une des revendications 1 à 5, caractérisée par des supports (27) réglables en hauteur pour ajuster l'angle d'inclinaison du tube rotatif (5).

7. Installation suivant l'une des revendications 1 à 6, caractérisée par un dispositif de barrage réglable (45 à 48) à l'extrémité de sortie du tube rotatif (5).

8. Installation suivant l'une des revendications 1 à 7, caractérisée en ce que le tube rotatif (5) est pourvu d'un capot chauffable (44).

9. Installation suivant la revendication 8, caractérisée en ce que le capot (44) est subdivisé en plusieurs zones.

10. Installation suivant l'une des revendications 1 à 9, caractérisée par un conduit qui est guidé suivant un circuit fermé et qui comporte le tube d'aspiration (53), le tube d'alimentation (38), un tube de dérivation (57) reliant le tube d'alimentation (38) à l'entrée de gaz (58), un ventilateur (55) et un élément de chauffage (56) pour le gaz de traitement.

11. Installation suivant la revendication 10, caractérisée par un conduit de déviation (59) qui court-circuite l'outil de coupe (17).

12. Installation suivant l'une des revendications 1 à 11, caractérisée en ce que la construction de support est réalisée sous la forme d'une plate-forme (1) d'un semi-remorque.

13. Installation suivant la revendication 12, caractérisée en ce que le puits de chargement (3) est agencé à l'arrière de la plate-forme et en ce qu'un dispositif élévateur-culbuteur (16) est agencé sur son côté arrière.

14. Installation suivant la revendication 13, caractérisée en ce que la plate-forme (1) présente dans la zone arrière une partie de fond (14) tirée vers le bas.

15. Installation suivant l'une des revendications 1 à 14, caractérisée en ce que le dispositif d'évacuation du corps de sortie (6) est équipé d'une vis d'évacuation (51) présentant un pas dégressif et un clapet de contre-pression et d'un conduit d'écoulement (65) agencé à l'endroit le plus profond.

16. Installation suivant la revendication 15, caractérisée en ce que la vis d'évacuation (51) peut être déployée latéralement à partir d'une position de transport dans une position de service.

17. Installation suivant l'une des revendications 12 à 16, caractérisée en ce qu'un appareil d'alimentation en énergie, qui comporte un brûleur (60), un générateur de vapeur, un élément chauffant (56) pour le milieu de traitement, un échangeur de chaleur pour de l'huile thermique ainsi que des éléments de commutation et de manoeuvre, est agencé sur le col de cygne (7) de la plate-forme (1) dans un logement séparé (8).

18. Installation suivant l'une des revendications 1 à 17, caractérisée par un corps (2) qui est relié à la construction de support (1) et entoure l'installation de désinfection et dont le fond est réalisé sous la forme d'une cuve.

19. Installation suivant l'une des revendications 1 à 18, caractérisée en ce que le brûleur (60) est relié au compartiment d'introduction (19) par un conduit d'aspiration d'air (61).

20. Installation suivant l'une des revendications 18 et 19, caractérisée en ce que le brûleur (60) est en communication avec l'intérieur du corps (2) par un conduit d'aspiration d'air (62).
